# FASCICULE DE BREVET EUROPEEN

(11) **EP 1 063 216 B1**
(45) Date de publication et mention de la délivrance du brevet: **16.04.2003**
(21) Numéro de dépôt: 00401746.3
(22) Date de dépôt: 19.06.2000
(51) Int. Cl.: C07C 15/107, C07C 2/66

(54) **Procédé de production de phénylalcanes utilisant un catalyseur à base d'une zéolithe de type structural EUO**
Verfahren zur Herstellung von Phenylalkanen unter Verwendung eines Katalysators vom Strukturtyp EUO
Process for the preparation of phenylalcanes using a catalyst based on a zeolite of the structural type EUO

(30) Priorité: 22.06.1999 FR 9907969
(43) Date de publication de la demande: 27.12.2000
(73) Titulaire: INSTITUT FRANCAIS DU PETROLE, 92852 Rueil-Malmaison Cedex (FR)
(72) Inventeur: Joly, Jean-François, 69006 Lyon (FR); Alario, Fabio, 92200 Neuilly sur Seine (FR); Merlen, Elisabeth, 92500 Rueil-Malmaison (FR); Magne-Drisch, Julia, 38200 Vilette de Vienne (FR)

(56) Documents cités:
- EP-A- 0 042 226
- EP-A- 0 051 318

## Description

### Domaine Technique

La présente invention concerne le domaine des procédés de production de phénylalcanes par alkylation du benzène au moyen d'au moins une oléfine comprenant au moins 9 atomes de carbone, ou comprenant au moins 14 atomes de carbone. Plus particulièrement, on utilisera par exemple une coupe hydrocarbonée comprenant au moins une oléfine ayant de 9 à 16 atomes de carbone ou de 10 à 14 atomes de carbone ou par exemple une coupe hydrocarbonée comprenant au moins une oléfine ayant de 14 à 20 atomes de carbone, en présence d'au moins un catalyseur comprenant une zéolithe de type structural EUO.

### Art Antérieur

Les phénylalcanes obtenus selon l'invention constituent des composés pour la formulation, après sulfonation, de détergents biodégradables.

Actuellement, les bases pour détergents biodégradables font largement appel aux alkylbenzènes linéaires. La production de ce type de composés est en croissance régulière. Une des propriétés principales recherchée pour ces composés, après l'étape de sulfonation, est, outre leur pouvoir détergent, leur biodégradabilité. Pour assurer une biodégradabilité maximale, le groupement alkyl doit être linéaire et long, la distance entre le groupe sulfonate et le carbone terminal de la chaîne linéaire doit être maximal. Les agents d'alkylation du benzène les plus intéressants sont constitués par les oléfines linéaires en C9-C16, et de préférence en C10-C14.

Les alkylbenzènes linéaires obtenus par alkylation du benzène au moyen d'oléfine(s) linéaire(s) sont préparés aujourd'hui par deux grands procédés. Le premier procédé utilise lors de l'étape d'alkylation du benzène, de l'acide fluorhydrique comme catalyseur acide. Le second utilise un catalyseur de type Friedel-Craft, en particulier à base de AlCl₃. Ces deux procédés conduisent à la formation de 2-, 3-, 4-, 5- et 6- phénylalcanes. Le principal inconvénient de ces procédés est lié à des contraintes d'environnement. Le premier procédé basé sur l'utilisation d'acide fluorhydrique pose des problèmes de sécurité sévères d'une part et de retraitement de déchets d'autre part. Le deuxième procédé pose le problème classique des procédés basés sur des catalyseurs de type Friedel-Craft, en l'occurrence le problème des rejets, en effet pour ce type de procédé, il est nécessaire de neutraliser les effluents par une solution basique en sortie de réacteur. A ces divers inconvénients s'ajoutent pour les deux procédés les difficultés liées à la séparation du catalyseur des produits de la réaction.

Ces diverses contraintes expliquent l'intérêt qu'il y a à mettre au point un procédé d'alkylation du benzène par les oléfines linéaires en présence d'un catalyseur solide.

L'art antérieur fait essentiellement état de l'utilisation de catalyseurs possédant des propriétés de sélectivité géométrique et conduisant à une sélectivité améliorée en 2- et 3-phénylalcane. Lesdits catalyseurs présentant des propriétés de sélectivité géométrique sont constitués de zéolithes. Ainsi le brevet US-A-4301317 a revendiqué toute une série de zéolithes dont : la cancrinite, la gmélinite, la mordénite, l'offrétite et la ZSM-12. Ces zéolithes sont, en particulier, caractérisées en ce que leur ouverture des pores est comprise entre environ 6 et environ 7 Å en diamètre (1 Å= 10-10 m).

Dans un brevet déposé par la demanderesse et publié sous le numéro FR-2697246, il est montré que l'on peut utiliser des catalyseurs à base de zéolithe Y désaluminée. Les zéolithes Y qui présentent une ouverture de pores voisine de 7,4 Å n'entrent pas dans le cadre du brevet US-A-4,301,317. Concernant l'emploi de zéolithes Y, le brevet EP-160.144 revendique l'utilisation de zéolithes Y mal cristallisées (cristallinité comprise entre 30 et 80 %),le brevet US-A-5,036,033 revendique l'utilisation de zéolithes Y riches en cations ammonium.

### Résumé

L'invention concerne un procédé de production d'au moins un composé choisi parmi les 2-, 3-, 4-, 5-, 6-phénylalcanes par alkylation du benzène au moyen d'au moins une oléfine contenant au moins 9 atomes de carbone, en présence d'un catalyseur solide zéolithique, caractérisé en ce que le catalyseur comprend une matrice et une zéolithe de type structural EUO, ladite zéolithe comprenant du silicium et au moins un élément T choisi dans le groupe formé par l'aluminium, le fer, le gallium et le bore, avec un rapport Si/T atomique global supérieur à 5 et caractérisé en ce que ladite alkylation est effectuée sous une pression d'environ 0,1 à 10 MPa, une température d'environ 30 à 400 °C, une vitesse spatiale horaire d'environ 0,5 à 200 h⁻¹ et un rapport molaire benzène/oléfine(s) d'environ 1:1 à 50:1. L'invention concerne tout particulièrement l'alkylation du benzène au moyen d'une oléfine linéaire, en vue de produire des phénylalcanes linaires.

### Intérêt

Nous avons découvert que l'utilisation de catalyseurs comprenant au moins une zéolithe de type structural EUO, au moins en partie sous forme acide, au moins une matrice, ladite zéolithe comprenant du silicium et au moins un élément T choisi dans le groupe formé par l'aluminium, le fer, le gallium et le bore, (de préférence l'aluminium et le bore), avec un rapport Si/T atomique supérieur à 5 présentent, en alkylation du benzène au moyen d'oléfine(s) telles que définies ci-devant des performances catalytiques supérieures à celles des catalyseurs décrits dans l'art antérieur. Ces nouveaux catalyseurs sont notamment à la fois très actifs, très sélectifs et très résistants à la désactivation.

### Description

Dans le cadre de la présente invention la charge hydrocarbonée employée pour effectuer l'alkylation du noyau benzénique peut contenir, outre au moins une oléfine telle que définie ci-devant, une ou plusieurs paraffines, un ou plusieurs autre(s) composé(s) aromatique(s), un ou plusieurs composé(s) polyoléfinique(s)(par exemple dioléfinique(s)), une ou plusieurs oléfine(s) non linéaire(s) mono- et ou poly-insaturée. Ces coupes peuvent aussi contenir une ou plusieurs alpha-oléfine(s) en quantité très variable depuis l'état de trace jusqu'à des quantités très fortes, voir largement majoritaires par rapport à ladite charge. Les oléfines peuvent être des oléfines linéaires ou ramifiées.

Le procédé selon la présente invention permet de produire, de préférence simultanément, au moins un composé choisi parmi les 2-, 3-, 4-, 5-, 6- phénylalcanes. Dans le cas où la charge comprend des oléfines linéaires, le procédé selon l'invention permet de produire en majeure partie des phénylalcanes linéaires. Dans le cas où la charge comprend des oléfines ramifiées, le procédé selon l'invention permet de produire en majeure partie des phénylalcanes ramifiés.

Le catalyseur utilisé dans la présente invention comporte au moins une zéolithe de type structural EUO, par exemple la zéolithe EU-1, au moins en partie sous forme acide, au moins une matrice (liant), ladite zéolithe comprenant du silicium et au moins un élément T choisi dans le groupe formé par l'aluminium, le fer, le gallium et le bore, de préférence l'aluminium et le bore, avec le rapport Si/T atomique global supérieur à 5. En outre, ce catalyseur mis en forme, par exemple sous forme de billes ou d'extrudés, présente une bonne résistance mécanique. La zéolithe EU-1 de type structural EUO, déjà décrite dans l'art antérieur, présente un réseau microporeux monodimensionnel, dont le diamètre des pores est de 4,1 x 5,7 Å (1 Å = 1 Angstrôm = 10-10 m) ("Atlas of Zeolite Structure Types", W.M. Meier et D.H. Olson, 4ème Edition, 1996). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Le mode de synthèse de la zéolithe EU-1 et ses caractéristiques physico-chimiques ont été décrits dans le brevet EP-B1- 42 226. Le brevet U.S.-4 640 829 concerne la zéolithe ZSM-50, qui, d'après l' "Atlas of Zeolite Structure Types ", W.M. Meier et D.H. Olson, 4^{ème} Edition, 1996, présente le même type structural EUO que la zéolithe EU-1. Ledit brevet présente un mode de synthèse de la ZSM-50 différent de celui décrit dans le brevet EP-B1-42 226 sur la zéolithe EU-1. La demande de brevet EP-A1-51 318 traite de la zéolithe TPZ-3, qui présente, d'après l' " Atlas of Zeolite Structure Types", W.M. Meier et D.H. Olson, 4^{ème} Edition, 1996, le même type structural EUO que la zéolithe EU-1, et de son utilisation en tant que catalyseur contenant la zéolithe telle quelle ou mise en forme. Dans ledit document la mise en forme de la zéolithe TPZ-3 est exemplifiée par la préparation de pastilles, obtenues par pastillage d'un mélange mécanique de poudres de zéolithe et de liant. Les pastilles comprennent la zéolithe TPZ-3, un liant et éventuellement au moins un élément choisi dans le groupe formé par le fer, le cobalt, le nickel, le cuivre, le zinc, le ruthénium, le rhodium, le palladium, le rhénium, l'osmium, l'iridium et le platine, sous forme de métal ou d'oxyde de métal.

De façon surprenante, il a été découvert par la demanderesse un catalyseur mis en forme, comprenant :
- au moins une zéolithe de type structural EUO, par exemple la zéolithe EU-1, au moins en partie et de préférence pratiquement totalement sous forme acide, contenant du silicium et au moins un élément T choisi dans le groupe formé par l'aluminium, le fer, le gallium et le bore, de préférence l'aluminium et le bore, et tel que le rapport atomique global Si/T est supérieur à 5,
- au moins une matrice (liant), par exemple l'alumine,
a des propriétés remarquables pour la production de phénylalcanes par alkylation du benzène au moyen de mono-oléfines.

La matrice (liant) consiste plus particulièrement en au moins un élément choisi dans le groupe formé par les argiles naturelles (comme par exemple le kaolin ou la bentonite), les argiles synthétiques, la magnésie, les alumines, les silices, les silice-alumines, l'oxyde de titane, l'oxyde de bore, la zircone, les phosphates d'aluminium, les phosphates de titane, les phosphates de zirconium, de préférence choisi parmi les éléments du groupe formé par les alumines et les argiles.

La zéolithe de type structural EUO, par exemple la zéolithe EU-1, comprise dans le catalyseur selon l'invention, est au moins en partie, de préférence pratiquement totalement, sous forme acide, c'est-à-dire sous forme hydrogène (H+), la teneur en sodium étant de préférence telle que le rapport atomique Na/T est inférieur à 0,5, de préférence inférieur à 0,1, de manière encore plus préférée inférieur à 0,02.

Selon une forme préférée de réalisation de l'invention on utilisera un catalyseur comprenant une zéolithe de type structural EUO dont la taille des cristaux est inférieure à 5 micromètres (µm), de manière préférée inférieure à 0,5 µm, et de manière encore plus préférée inférieure à 0,2 µm. Ces cristaux ou cristallites sont souvent regroupés en agrégats présentant une granulométrie telle que la valeur de Dv,90 est inférieure ou égale à 500 µm, souvent inférieure à 400 µm et le plus souvent inférieure à 200 µm et de manière plus préférée inférieure à 50 µm. La taille des agrégats est déterminée par granulométrie à diffraction laser. Cette mesure est réalisée sur la poudre de zéolithe mise en suspension dans de l'eau. Après une première mesure, la suspension est soumise à des ultrasons pendant trente secondes puis une nouvelle mesure est réalisée. Les ultrasons utilisés sont caractérisés par une puissance de 50 W et une fréquence de 50 kHz. Cette procédure est répétée jusqu'à ce que le résultat ne varie plus (à ± 5 %). La distribution en taille des agrégats définie en volume est calculée à partir des signaux lumineux collectés par les détecteurs et avec la théorie de Fraunhofer. On définit Dv,X comme étant le diamètre de la sphère équivalente telle que X% en volume des agrégats a une taille inférieure audit diamètre. Ces caractéristiques seront obtenues directement lors de la synthèse de la zéolithe et/ou par toute méthode permettant de diminuer la taille des agrégats comme par exemple le broyage post-synthèse ou encore un malaxage adéquat avant mise en forme.

Le catalyseur utilisé dans le procédé selon l'invention contient plus particulièrement :
- en teneur pondérale, de 1 à 95% et de préférence de 3 à 90% bornes incluses, et de manière encore plus préférée de 5 à 85% d'au moins une zéolithe de type structural EUO, par exemple la zéolithe EU-1, au moins en partie sous forme acide, comprenant du silicium et au moins un élément T choisi dans le groupe formé par l'aluminium, le fer, le gallium et le bore, de préférence l'aluminium et le bore, dont le rapport Si/T atomique global est supérieur à 5.
- au moins une matrice ou liant, assurant le complément à 100% dans le catalyseur.

Ledit catalyseur présente de préférence une valeur d'écrasement en lit, déterminée selon la méthode Shell (SMS 1471-74) et caractérisant sa résistance mécanique, supérieure à 0,5 MPa.

Le catalyseur utilisé dans le procédé selon l'invention peut être préparé par toute méthode connue de l'homme du métier et en particulier par celles décrites dans l'art antérieur concernant les catalyseur contenant au moins une zéolithe de type structural EUO et en particulier la zéolithe EU1, ZSM-50 et TPZ-3.

Pour préparer le catalyseur utilisé dans le procédé selon l'invention, on opère par exemple d'abord un traitement de la zéolithe de type structural EUO, par exemple la zéolithe EU-1, brute de synthèse, selon toute méthode connue de l'homme du métier, par exemple on procède à une étape de calcination sous flux d'air sec, qui a pour but d'éliminer le structurant organique occlus dans la microporosité de la zéolithe, puis on procède à au moins une étape d'échange ionique par exemple par au moins une solution de NH4NO3, de manière à éliminer au moins en partie, de préférence pratiquement totalement, tout cation alcalin, en particulier le sodium, présent en position cationique dans la zéolithe.
On poursuit la préparation du catalyseur en mélangeant la matrice et la zéolithe préparée précédemment puis on met en forme. La mise en forme du catalyseur selon l'invention est généralement telle que le catalyseur est de préférence sous forme d'extrudés ou de billes, en vue de son utilisation. Les conditions de mise en forme de la zéolithe, le choix de la matrice, éventuellement le broyage préalable de la zéolithe, le procédé de peptisation, l'ajout d'agents porogènes, le temps de malaxage, la pression d'extrusion si le catalyseur est mis sous forme d'extrudés, la vitesse et le temps de séchage, sont déterminés, pour chaque matrice, selon les règles bien connues de l'homme de métier, de manière à obtenir un catalyseur de préférence sous forme d'extrudés ou de billes.

La préparation du catalyseur se poursuit généralement par une calcination, habituellement à une température comprise entre 250°C et 600°C bornes incluses, de préférence précédée d'un séchage, par exemple à l'étuve, à une température généralement comprise entre la température ambiante et 250°C bornes incluses, de préférence entre 40°C et 200°C bornes incluses. Ladite étape de séchage est de préférence menée pendant la montée en température nécessaire pour effectuer ladite calcination.

La mise en forme de la zéolithe de type structural EUO utilisée dans le procédé selon l'invention, par exemple la zéolithe EU-1, peut être effectuée sur la zéolithe brute de synthèse, c'est-à-dire contenant le structurant organique et des cations alcalins, généralement le sodium. Dans ce cas l'étape de calcination sous flux d'air sec, ayant pour but d'éliminer le structurant organique, et les étapes d'échanges ioniques par au moins une solution de NH₄NO₃ sont réalisées sur le catalyseur mis en forme comprenant la zéolithe et la matrice.

Selon une variante préférée du procédé de l'invention, on fait réagir, dans une zone de réaction, du benzène avec une charge renfermant au moins une oléfine, par exemple, linéaire, au contact d'un catalyseur comprenant une zéolithe de type structural EUO ayant les caractéristiques définies précédemment (réaction d'alkylation), puis on fractionne le produit obtenu de manière à recueillir séparément une première fraction renfermant du benzène non converti, une deuxième fraction renfermant au moins une oléfine par exemple, linéaire, initialement présente dans la charge (non convertie), une troisième fraction renfermant des 2-, 3-, 4-, 5- et 6- phénylalcanes et une quatrième fraction renfermant au moins un poly-alkylbenzène (ou fraction poly-alkylbenzène), celle ci étant ensuite, le plus souvent au moins en partie recyclée vers ladite zone de réaction où elle réagit avec du benzène au contact dudit catalyseur, afin d'être au moins en partie transalkylée (réaction de transalkylation), et on recueille un mélange de 2-, 3-, 4-, 5- et 6-phénylalcanes.

Cette variante de l'invention est donc notamment caractérisée par le fait que les réactions d'alkylation et de transalkylation ont lieu conjointement dans la même zone de réaction (c'est-à-dire) dans le même réacteur en présence du même catalyseur. De manière préférée, la première fraction renfermant du benzène non converti est au moins en partie recyclée vers ladite zone de réaction. De même, de manière préférée, la deuxième fraction renfermant au moins une oléfine linéaire non convertie est au moins en partie recyclée vers ladite zone de réaction.

La partie recyclée de la quatrième fraction contenant en majeure partie en général au moins un dialkylbenzène est de préférence sensiblement exempte d'alkylaromatiques lourds qui peuvent être éliminés par fractionnement.

Selon une autre variante de réalisation de l'invention au moins une partie de la quatrième fraction contenant un polyakylbenzène, comprenant en majeure partie en général au moins un dialkylbenzène et de préférence sensiblement exempte d'alkyl aromatiques lourds qui peuvent être éliminés par fractionnement, peut être envoyée dans une deuxième zone de réaction, différente de la zone d'alkylation du benzène contenant un catalyseur structural EUO, ladite deuxième zone de réaction contenant un catalyseur de transalkylation (par exemple une zéolithe de type structural EUO ou tout autre catalyseur de transalkylation bien connus de l'homme du métier) et étant maintenue dans des conditions permettant la formation de monoalkylbenzene par réaction entre ce ou ces polyakylbenzène(s) et du benzène frais ou provenant au moins en partie de la première fraction obtenue lors du fractionnement de l'effluent du mélange sortant de la zone d'alkylation du benzène contenant un catalyseur à base de zéolithe de type structural EUO.

Le procédé selon la présente invention peut être effectué, pour l'étape d'alkylation, à une température comprise entre 30 et 400 °C, sous une pression de 0,1 à 10 MPa, avec un débit d'hydrocarbures liquides (vitesse spatiale horaire) d'environ 0,5 à 200 volumes par volume de catalyseur et par heure et avec un rapport molaire benzène/oléfine(s) compris entre 1:1 et 50:1.

Dans la mise en oeuvre de l'invention qui comprend une deuxième étape distincte de transalkylation, la deuxième étape peut être effectuée à une température comprise entre environ 100 et 500°C, de préférence entre 150 et 400°C, sous-une pression comprise entre environ 1,5 et 10 MPa (de préférence de 2 à 7 MPa), avec un débit d'hydrocarbures liquides (vitesse spatiale) d'environ 0,5 à 5 volumes par volume de catalyseur et par heure, et avec un rapport molaire benzène/polyalkylbenzènes d'environ 2 : 1 à 50 : 1.

### Exemple 1

La matière première utilisée est une zéolithe de type structural EUO, la zéolithe EU-1, brute de synthèse, comprenant le structurant organique, du silicium et de l'aluminium, possédant un rapport atomique Si/AI global égal à 13,6 , une teneur pondérale en sodium par rapport au poids en zéolithe EU-1 sèche d'environ 1,5%, correspondant à un rapport atomique Na/AI de 0,6.

Cette zéolithe EU-1 subit tout d'abord une calcination dite sèche à 550°C sous flux d'air durant 6 heures. Puis le solide obtenu est soumis à trois échanges ioniques dans une solution de NH₄NO₃ 10N, à environ 100°C pendant 4 heures pour chaque échange.

A l'issue de ces traitements, la zéolithe EU-1 sous forme NH₄ a un rapport Si/AI atomique global égal à 18,3 , une teneur pondérale en sodium par rapport au poids de zéolithe EU-1 sèche de 50 ppm, correspondant à un rapport atomique Na/AI de 0,003, une surface spécifique mesurée par la méthode BET de 407 m²/g et un volume poreux, à l'azote, mesuré à -196°C et à P/PO = 0,15, de 0,16 cm3 d'azote liquide par gramme. Dans la zéolithe EU-1, 100% des atomes d'aluminium sont en coordinence tétraédrique, d'après l'analyse par RMN de l'aluminium 27.

La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 60 % de zéolithe EU-1 sous forme H et 40 % d'alumine. Le diamètre des pores du catalyseur ainsi préparé, mesuré par porosimétrie au mercure, se situe entre 40 et 90 Å, la distribution des diamètres de ces mésopores étant monomodale et centrée sur 70 Å. La valeur de l'écrasement lit, obtenu selon la méthode Shell, est de 1,1 MPa.

Le catalyseur ainsi préparé est utilisé en alkylation du benzène par le dodécène-1. Les conditions opératoires suivantes sont utilisées :
- Température : 220°C
- Pression opératoire : 4 MPa
- VVH : 1 l/l/h
- Rapport molaire benzène/dodécène-1 : 5,4 mol/mol.

Dans de telles conditions opératoires, la conversion exprimée par rapport au dodécène-1 est de 53,4 %. Les résultats de sélectivités en 2-, 3-, 4-, 5- et 6- phénylalcane, obtenus après réaction, sont rassemblés dans le tableau 1.

**Tableau 1**

| Composés | Sélectivités |
|---|---|
| 2- Phénylcalcane | 17,13 |
| 3- Phénylcalcane | 5,69 |
| 4- Phénylcalcane | 3,69 |
| 5- Phénylcalcane | 3,13 |
| 6- Phénylcalcane | 2,84 |
| branchés | 20,92 |

## Revendications

1. Procédé de production d'au moins un composé choisi parmi les 2-, 3-, 4-, 5-, 6-phénylalcanes par alkylation du benzène au moyen d'au moins une oléfine contenant au moins 9 atomes de carbone, en présence d'un catalyseur solide zéolithique, **caractérisé en ce que** le catalyseur comprend une matrice et une zéolithe de type structural EUO, ladite zéolithe comprenant du silicium et au moins un élément T choisi dans le groupe formé par l'aluminium, le fer, le gallium et le bore, avec un rapport Si/T atomique global supérieur à 5 et **caractérisé en ce que** ladite alkylation est effectuée sous une pression d'environ 0,1 à 10 MPa, une température d'environ 30 à 400 °C, une vitesse spatiale horaire d'environ 0,5 à 200 h⁻¹ et un rapport molaire benzène/oléfine(s) d'environ 1:1 à 50:1.

2. Procédé selon la revendication 1 dans lequel la production de 2-, 3-, 4-, 5-, 6-phénylalcanes est simultanée.

3. Procédé selon l'une des revendications 1 à 2 dans lequel l'oléfine est linéaire.

4. Procédé selon l'une des revendications 1 à 2 dans lequel l'oléfine est ramifiée.

5. Procédé selon l'une des revendications 1 à 4 dans lequel le catalyseur contient une zéolithe de type structural EUO dont la taille des cristaux est inférieure à 5 micromètres (µm).

6. Procédé selon la revendication 5 tel que les cristaux sont regroupés en agrégats présentant une granulométrie telle que la valeur de Dv,90 est inférieure ou égale à 500 µm.

7. Procédé selon l'une des revendications 1 à 6 dans lequel l'oléfine contient de 9 à 16 atomes de carbone par molécule.

8. Procédé selon l'une des revendications 1 à 7 dans lequel l'oléfine contient de 14 à 20 atomes de carbone par molécule.

9. Procédé selon l'une des revendications 1 à 8 dans lequel la matrice est choisie dans le groupe formé par les argiles, les alumines, la silice, la magnésie, le zircone, l'oxyde de titane, l'oxyde de bore et leurs combinaisons.

10. Procédé selon l'une des revendications 1 à 9 **caractérisé en ce que** le catalyseur est mis en forme sous forme de billes ou d'extrudés.

11. Procédé selon l'une des revendications 1 à 10 **caractérisé en ce que** le catalyseur présente une résistance mécanique telle que la valeur de l'écrasement en lit est supérieure à 0,5 MPa.

12. Procédé selon l'une des revendications 1 à 11 dans lequel la zéolithe de type structural EUO est la zéolithe EU-1.

13. Procédé selon l'une des revendications 1 à 12 **caractérisé en ce que** l'élément T du catalyseur est choisi dans le groupe formé par l'aluminium et le bore.

14. Procédé selon l'une des revendications 1 à 13 **caractérisé en ce que** la matrice du catalyseur est de l'alumine.

15. Procédé selon l'une des revendications 1 à 14 **caractérisé en ce que** la zéolithe du catalyseur est au moins en partie sous forme acide avec un rapport atomique Na/T inférieur à 0,5.

16. Procédé selon l'une des revendications 1 à 15 **caractérisé en ce que** le catalyseur contient de 1% à 95% en poids d'au moins une zéolithe de type structural EUO par rapport au poids total de catalyseur.

17. Procédé selon l'une des revendications 1 à 16, dans lequel le produit obtenu à l'issue de la zone d'alkylation est fractionné en une première fraction renfermant du benzène non converti, une seconde fraction renfermant au moins une oléfine non convertie, une troisième fraction renfermant les phénylalcanes et une quatrième fraction renfermant au moins un polyalkylbenzène, ladite quatrième fraction étant au moins en partie recyclée vers la zone d'alkylation.

18. Procédé selon l'une des revendications 1 à 16, dans lequel le produit obtenu à l'issue de la zone d'alkylation est fractionné en une première fraction renfermant du benzène non converti, une seconde fraction renfermant au moins une oléfine non convertie, une troisième fraction renfermant les phénylalcanes et une quatrième fraction renfermant au moins un polyalkylbenzène, ladite quatrième fraction étant au moins en partie traitée dans une deuxième zone de réaction, différente de la zone d'alkylation du benzène contenant un catalyseur structural EUO, ladite deuxième zone de réaction contenant un catalyseur de transalkylation, et étant maintenue dans des conditions permettant la formation de monoalkylbenzene par réaction entre ce ou ces polyakylbenzène(s) et du benzène.

19. Procédé selon la revendication 18 telle que la zone de transalkylation est mise en oeuvre à une température comprise entre environ 100 et 500°C, sous une pression comprise entre environ 1,5 et 10 MPa, avec un débit d'hydrocarbures liquides (vitesse spatiale) d'environ 0,5 à 5 volumes par volume de catalyseur et par heure, et avec un rapport molaire benzène/polyalkylbenzènes d'environ 2 : 1 à 50 : 1

20. Procédé selon l'une des revendications 17 à 19, dans lequel la première fraction est au moins en partie recyclée vers la zone d'alkylation.

21. Procédé selon l'une des revendications 17 à 20, dans lequel la deuxième fraction est au moins en partie recyclée vers la zone d'alkylation.

## Claims

1. A process for producing at least one compound selected from 2-, 3-, 4-, 5- and 6-phenylalkanes by alkylating benzene using at least one olefin containing at least 9 carbon atoms per molecule, in the presence of a solid zeolitic catalyst, **characterized in that** the catalyst comprises a matrix and a zeolite with structure type EUO, said zeolite comprising silicon and at least one element T selected from the group formed by aluminium, iron, gallium and boron, with an overall Si/T atomic ratio of more than 5; and **characterized in that** said alkylation is carried out at a pressure in the range 0.1 to 10 MPa, a temperature in the range 30°C to 400°C, with an hourly space velocity of 0.50 to 200 h⁻¹, and a benzene/olefin(s) mole ratio in the range 1:1 to 50:1.

2. A process according to claim 1, in which the production of 2-, 3-, 4-, 5- and 6-phenylalkanes is simultaneous.

3. A process according to claim 1 or claim 2, in which the olefin is linear.

4. A process according to claim 1 or claim 2, in which the olefin is branched.

5. A process according to any one of claims 1 to 4, in which the catalyst contains a zeolite with structure type EUO the crystal size of which is less than 5 micrometers (µm).

6. A process according to claim 5, in which the crystals are arranged in aggregates with a grain size such that the value of Dv,90 is 500 µm or less.

7. A process according to any one of claims 1 to 6, in which the olefin contains 9 to 16 carbon atoms per molecule.

8. A process according to any one of claims 1 to 7, in which the olefin contains 14 to 20 carbon atoms per molecule.

9. A process according to any one of claims 1 to 8, in which the matrix is selected from the group formed by clays, aluminas, silica, magnesia, zirconia, titanium oxide, boron oxide and combinations thereof.

10. A process according to any one of claims 1 to 9, **characterized in that** the catalyst is formed into beads or extrudates.

11. A process according to any one of claims 1 to 10, **characterized in that** the mechanical strength of the catalyst is such that the bed crush strength is more than 0.5 MPa.

12. A process according to any one of claims 1 to 11, in which the zeolite with structure type EUO is EU-1 zeolite.

13. A process according to any one of claims 1 to 12, **characterized in that** the element T in the catalyst is selected from the group formed by aluminium and boron.

14. A process according to any one of claims 1 to 13, **characterized in that** the matrix of the catalyst is alumina.

15. A process according to any one of claims 1 to 14, **characterized in that** the zeolite of the catalyst is at least partially in its acid form with an Na/T atomic ratio of less than 0.5.

16. A process according to any one of claims 1 to 15, **characterized in that** the catalyst contains 1% to 95% by weight of at least one zeolite with structure type EUO with respect to the total catalyst weight.

17. A process according to any one of claims 1 to 16, in which the product obtained from the alkylation zone is fractionated into a first fraction comprising unconverted benzene, a second fraction comprising at least one unconverted olefin, a third fraction comprising phenylalkanes and a fourth fraction comprising at least one polyalkylbenzene, at least a portion of said fourth fraction being recycled to the alkylation zone.

18. A process according to any one of claims 1 to 16, in which the product obtained from the alkylation zone is fractionated into a first fraction comprising unconverted benzene, a second fraction comprising at least one unconverted olefin, a third fraction comprising phenylalkanes and a fourth fraction comprising at least one polyalkylbenzene, at least a portion of said fourth fraction being treated in a second reaction zone, different from the zone for alkylating benzene containing a catalyst with structure type EUO, said second reaction zone containing a transalkylation catalyst and being maintained under conditions for forming monoalkylbenzene by reaction between the polyalkylbenzene(s) and benzene.

19. A process according to claim 18, in which the transalkylation zone is operated at a temperature in the range about 100°C to 500°C, at a pressure in the range about 1.5 to 10 MPa, with a liquid hydrocarbon flow rate (space velocity) of about 0.5 to 5 volumes per volume of catalyst per hour, and with a benzene/polyalkylbenzene mole ratio of about 2:1 to 50:1.

20. A process according to any one of claims 17 to 19, in which at least a portion of the first fraction is recycled to the alkylation zone.

21. A process according to any one of claims 17 to 20, in which at least a portion of the second fraction is recycled to the alkylation zone.

## Patentansprüche

1. Verfahren zur Herstellung von wenigstens einer Verbindung, gewählt unter den 2-, 3-, 4-, 5-, 6-Phenylalkanen durch Alkylierung des Benzols mittels wenigstens eines Olefins, das wenigstens 9 Kohlenstoffatome enthält, in Gegenwart eines festen zeolithischen Katalysators, **dadurch gekennzeichnet, dass** der Katalysator eine Matrix und einen Zeolith vom Strukturtyp EUO umfasst, welcher Zeolith Silizium und wenigstens ein Element T umfasst, das aus der durch Aluminium, Eisen, Gallium und Bor gebildeten Gruppe gewählt ist, mit einem Si/T Gesamtatomverhältnis über 5 und **dadurch gekennzeichnet, dass** die Alkylierung unter einem Druck von etwa 0,1 bis 10 MPa, einer Temperatur von etwa 30 bis 400°C und einer stündlichen Raumgeschwindigkeit von etwa 0,5 bis 200 h⁻¹ und einem molaren Verhältnis Benzol/Olefin(e) von etwa 1:1 bis 50 : 1 durchgeführt wird.

2. Verfahren nach Anspruch 1, bei dem die Herstellung von 2-, 3-, 4-, 5-, 6-Phenylalkanen simultan gemacht ist.

3. Verfahren nach einem der Ansprüche 1 bis 2, bei dem das Olefin linear ist.

4. Verfahren nach einem der Ansprüche 1 bis 2, bei dem das Olefin verzweigt ist.

5. Verfahren nach einem der Ansprüche 1 bis 4, bei dem der Katalysator einen Zeolith vom Strukturtyp EUO enthält, dessen Größe der Kristalle kleiner als 5 Mikrometer (µm) liegt.

6. Verfahren nach Anspruch 5, derart, dass die Kristalle zu Aggregaten vereint sind, welche eine Granulometrie derart aufweisen, dass der Dv,90-Wert kleiner oder gleich 500 µm ist.

7. Verfahren nach einem der Ansprüche 1 bis 6, bei dem das Olefin 9 bis 16 Kohlenstoffatome pro Molekül enthält.

8. Verfahren nach einem der Ansprüche 1 bis 7, bei dem das Olefin 14 bis 20 Kohlenstoffatome pro Molekül enthält.

9. Verfahren nach einem der Ansprüche 1 bis 8, bei dem die Matrix gewählt wird aus der Gruppe, die gebildet wird durch die Tone, die Aluminiumoxyde, Siliziumoxyd, Magnesiumoxyd, Zirkoniumoxyd, Titanoxyd, Boroxyd und deren Kombinationen.

10. Verfahren nach einem der Ansprüche 1 bis 9, **dadurch gekennzeichnet, dass** der Katalysator in Form von Kugeln oder Extrudaten geformt wird.

11. Verfahren nach einem der Ansprüche 1 bis 10, **dadurch gekennzeichnet, dass** der Katalysator eine mechanische Resistenz derart aufweist, dass der Bruchwert im Bett über 0,5MPa liegt.

12. Verfahren nach einem der Ansprüche 1 bis 11, bei dem der Zeolith vom Strukturtyp EUO der EU-1-Zeolith ist.

13. Verfahren nach einem der Ansprüche 1 bis 12, **dadurch gekennzeichnet, dass** das Element T des Katalysators gewählt wird in der durch Aluminium und Bor gebildeten Gruppe.

14. Verfahren nach einem der Ansprüche 1 bis 13, **dadurch gekennzeichnet, dass** die Matrix des Katalysators Aluminiumoxyd ist.

15. Verfahren nach einem der Ansprüche 1 bis 14, **dadurch gekennzeichnet, dass** der Zeolith des Katalysators wenigstens teilweise in Säureform mit einem Atomverhältnis Na/T unter 0,5 vorliegt.

16. Verfahren nach einem der Ansprüche 1 bis 15, **dadurch gekennzeichnet, dass** der Katalysator von einem Gewichtsprozent bis 95 Gewichtsprozent wenigstens eines Zeolithen vom Strukturtyp EUO im Verhältnis zum Gesamtgewicht des Katalysators enthält.

17. Verfahren nach einem der Ansprüche 1 bis 16, bei dem das aus der Alkylierungszone erhaltene Produkt in eine erste Fraktion fraktioniert wird, die nicht umgewandeltes Benzol einschließt, wobei eine zweite Fraktion wenigstens ein nicht umgewandeltes Olefin einschließt, eine dritte Fraktion die Phenylalkane einschließt und eine vierte Fraktion wenigstens ein Polyalkylbenzol einschließt, wobei die vierte Fraktion wenigstens teilweise zur Alkylierungszone recykliert wird.

18. Verfahren nach einem der Ansprüche 1 bis 16, bei dem das aus der Alkylierungszone erhaltene Produkt in eine erste Fraktion fraktioniert wird, welche nicht umgewandeltes Benzol einschließt, wobei eine zweite Fraktion wenigstens ein nicht umgewandeltes Olefin einschließt, eine dritte Fraktion die Phenylalkane einschließt und eine vierte Fraktion wenigstens ein Polyalkylbenzol einschließt, wobei die vierte Fraktion wenigstens teilweise in einer zweiten Reaktionszone behandelt wird, welche verschieden von der Zone zur Alkylierung des Benzols ist, die einen Katalysator mit EUO-Struktur enthält, wobei die zweite Reaktionszone einen Transalkylierungskatalysator enthält und unter Bedingungen gehalten wird, die die Bildung von Monoalkylbenzol durch Reaktion zwischen diesem oder diesen Polyalkylbenzol(en) und Benzol ermöglichen.

19. Verfahren nach Anspruch 18, derart, dass die Transalkylierungszone bei einer Temperatur zwischen etwa 100 und 500°C unter einem Druck zwischen etwa 1,5 und 10 MPa mit einem Flüssig-Kohlenwasserstoff-Durchsatz (Raumgeschwindigkeit) von etwa 0,5 bis 5 Volumen pro Katalysatorvolumen und pro Stunde und mit einem molaren Verhältnis Benzol/Polyalkylbenzole von etwa 2 : 1 bis 50 : 1 eingesetzt wird.

20. Verfahren nach einem der Ansprüche 17 bis 19, bei dem die erste Fraktion wenigstens teilweise zur Alkylierungszone recykliert wird.

21. Verfahren nach einem der Ansprüche 17 bis 20, bei dem die zweite Fraktion wenigstens teilweise zur Alkylierungszone recykliert wird.
